# EUROPÄISCHE PATENTSCHRIFT

(11) **EP 3 121 596 B1**
(45) Veröffentlichungstag und Bekanntmachung des Hinweises auf die Patenterteilung: **20.09.2017**
(21) Anmeldenummer: 16179723.8
(22) Anmeldetag: 15.07.2016
(51) Int. Cl.: G01N 33/28, G01N 21/64, G01N 15/06, G01N 21/77

(54) **VERFAHREN ZUR ERMITTLUNG DES GEHALTS AN CATALYST FINES IN EINER PROBE EINES SCHWERÖLS**
METHOD FOR DETERMINING THE CONTENT OF CATALYST FINES IN A SAMPLE OF A HEAVY OIL
PROCÉDÉ DE DÉTERMINATION DE TENEUR EN FINES PARTICULES CATALYTIQUES DANS UN ÉCHANTILLON D'HUILE LOURDE

(30) Priorität: 22.07.2015 EP 15177929
(43) Veröffentlichungstag der Anmeldung: 25.01.2017
(73) Patentinhaber: CM Technologies GmbH, 35337 Elmshorn (DE)
(72) Erfinder: Jeske, Andreas, 25335 Elmshorn (DE); Winkler, Matthias, 25348 Glückstadt (DE)
(74) Vertreter: Meyer, Ludgerus

(56) Entgegenhaltungen:
- WO-A1-2014/001168
- US-B1- 7 286 633

## Beschreibung

Die Erfindung bezieht sich auf ein Verfahren zur Ermittlung des Gehalts an Catalyst Fines in einer Probe eines Schweröls, insbesondere eines Kraftstoffes für Schiffsmotoren.

Auf Seeschiffen, insbesondere für den internationalen Gütertransport, sind die Antriebsaggregate in der Regel als Dieselmotoren ausgeführt, denen als Brennstoff Schweröle zugeführt werden. Diese sind hauptsächlich aus Rückständen der Erdölverarbeitung hergestellt. Während der Destillation von Rohöl in Erdölraffinerien werden beim sogenannten katalytischen Cracken Katalysatoren verwendet, die Catalyst Fines (Catfines) enthalten. Dabei handelt es sich hauptsächlich um Aluminium-Siliziumoxide (Al₂O₃ und SiO₂). Diese sind sehr hart und abrasiv. Sie verbleiben nach dem Cracken zum Teil in den Rückstandsölen, die dann als Kraftstoffe für die Schiffsdieselmotoren verwendet werden und stellen damit eine erhebliche Gefahr für verschiedene Bauteile des Einspritzsystems, den Kolbenringen und den Zylinderbuchsen dar. Vor der Verwendung derartiger Kraftstoffe ist daher eine gründliche Aufbereitung erforderlich, die auch eine Filtration des Kraftstoffs beinhaltet.

In der ISO Norm 8217 ist für die Lieferung von Schweröl ein maximaler Gehalt von 60 ppm Catfines vorgeschrieben. Dagegen schreiben Motorenhersteller einen maximalen Gehalt von 10 -15 ppm Catfines vor Eintritt in den Motor vor.

Neben der Filtration des Kraftstoffs kann die Abtrennung von Catfines aus den Schwerölen auch mittels Zentrifugen vorgenommen werden. Ein entsprechendes Verfahren und ein System zur Abtrennung von Catalyst Fines aus einem Ölstrom ist in der WO 2014/001168 A1 angegeben. In Abhängigkeit von dem durch ein NMR-Verfahren erfassten Catfines-Gehalt im Schweröl erfolgt mittels der Zentrifuge eine mehr oder weniger starke Reinigung des Schweröls.

Ein Verfahren zur Messung der Konzentration von Catalyst Fines mittels Röntgenfluoreszenz ist aus US 7,286,633B1 bekannt. Ein solches Verfahren kann zwar den Gehalt am Catalyst Fines im Schweröl reduzieren, jedoch ist die Feststellung des Gehalts an Catalyst Fines im Schweröl mittels einer NMR-Spektroskopie auf einem Seeschiff normalerweise nicht möglich und zu aufwendig.

Der Erfindung liegt daher die Aufgabe zugrunde, ein einfaches Verfahren zur Ermittlung des Gehalts an Catalyst Fines in einem Schweröl, insbesondere eines Kraftstoffs für Schiffsmotoren, anzugeben, das vor Ort auf einem Schiff verwendbar ist und eine praxisgerechte Aussage über den Gehalt an Catalyst Fines in einem Schweröl liefert.

Diese Aufgabe wird durch die im Anspruch 1 angegebene Erfindung gelöst. Vorteilhafte Weiterbildungen der Erfindung sind in Unteransprüchen angegeben.

Gemäß dem erfindungsgemäßen Verfahren wird eine verdünnte Probe eines Schweröls mit Kaliumhydroxid zur oberflächlichen Ionisierung von Aluminium aus den Catfines vermischt. Dieses ionisierte dreiwertige Aluminium-Ion verhält sich in wässrigen Lösungen amphoter: es bildet mit wässrigen Bestandteilen pH-Wert-abhängig unterschiedliche Komplexe. Durch die Anregung dieser Komplexe mittels UV-Licht und einer speziellen Detektionswellenlänge kann die Schwächung der Strahlung im Spektrum erkannt werden.

Da das Verhältnis zwischen Aluminiumoxide und Siliziumoxide in den Catfines ungefähr gleich ist, kann allein aus der Feststellung des Aluminiumgehalts der gesamte Catfines-Gehalt in einer Probe des Schweröls ermittelt werden.

Insbesondere wird bei dem erfindungsgemäßen Verfahren zunächst eine Probe eines Schweröls mit einem ersten Reagenz, das hauptsächlich aus aliphatischen Kohlenwasserstoffen besteht und üblicherweise als WBC-Verdünner bezeichnet wird, verdünnt und intensiv vermischt. Dadurch wird die Zugänglichkeit zu den Aluminiumsilikaten der Catfines gesichert. Ein Teil der Mischung aus dem ersten Behälter wird in einen zweiten eine Mischung von Methanol und KOH-Ethanol (kaliumhydroxidhaltiges Ethanol)als zweites Reagenz enthaltenden zweiten Behälter eingebracht und damit intensiv vermischt, um eine oberflächliche Ionisierung des Aluminiums aus den Catfines zu erreichen und eine Pufferung im Bereich von pH 10 - 14 zu erzielen.

Ein Teil des Inhalts des zweiten Behälters wird dann über einen Filter in einen dritten Behälter überführt, in dem die Feststellung des Gehalts an Catalyst Fines in der Probe bei UV-Lichtanregung durch Ermittlung der Stärke der sich ergebenden Fluoreszenzstrahlung visuell oder mit Hilfe eines Photometers erfasst wird.

Die Quantifizierung erfolgt somit im basischen Bereich durch Bildung von Komplexe wie folgt:

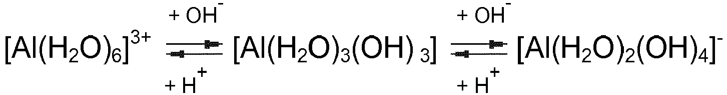

Die Formel beschreibt ein pH-Wert- abhängiges amphoteres Verhalten von Aluminium-Ionen in Wasser mit der Folge der Komplexbildung.
Links: dreiwertiger Komplex im stark sauren Bereich
Mitte: neutraler Komplex bei pH = 7
Rechts: einfach negativ geladener Komplex im stark basischen Bereich

Als Verdünner für das Schweröl wird vorzugsweise ein WBC6-Verdünner verwendet, bei dem es sich um einen Standardverdünner in Form eines Universalkaltreinigers handelt, der aus aliphatischen Kohlenwasserstoffen mit n-Alkanen, C10-C13- Verbindungen, iso-Alkanen und Cycloalkanen besteht.

Das zweite Reagenz ist vorzugsweise eine 50:50 Methanol/KOH-Ethanol-Mischung, der vorzugsweise in kleiner Menge von 0,1 - 0,2% ein oberflächenaktives Tensid als alkalisches Reinigungskonzentrat mit pH 6,2 - 7,2 beigefügt ist.

Die Entnahme aus dem zweiten Behälter erfolgt vorzugsweise mit einer 5 ml Kolbenspritze, der ein mit Kaliumhydroxid vorgespülter Filter vorgesetzt ist.

Die Durchmischung der Probe und der Reagenzien erfolgt vorzugsweise mittels eines Vortexmischers.

Zur Beschleunigung des Verfahrens kann die Vermischung auch mit erhöhter Temperatur unter Verwendung von Ultraschall durchgeführt werden.

Die Feststellung des Gehalts in Catalyst Fines in der Probe erfolgt vorzugsweise durch Anregung mittels einer UV-Diode bei einer Wellenlänge von 365 nm. Die sich ergebende Fluoreszenzstrahlung kann bei 470 nm visuell erfasst werden oder vorzugsweise mittels eines Photometers ausgewertet werden.

Das erfindungsgemäße Verfahren kann vor Ort auf einem Schiff mit einfachen Mitteln durchgeführt werden, so dass eine ständige Kontrolle der Qualität des Schweröls möglich ist, sofern nicht bereits bei Bunkerung des Schweröls eine entsprechende Prüfung durchgeführt wurde. Da Catfines jedoch die Tendenz haben, in einem Schwerölbunker einen Bodensatz zu bilden, ist es von Vorteil, die Analyse des Schweröls während einer Fahrt mehrfach zu wiederholen.

In einem beispielhaft durchgeführten Verfahren wird Schweröl im Verhältnis von 1:10, vorzugsweise 2 ml Schweröl und 20 ml WBC-Verdünner, in einem ersten Behälter mittels eines Vortexmischers für 1- 3 Minuten gemischt. Mit einer Kolbenspritze werden 7 ml einer 50:50 Mischung aus Methanol und KOH-Ethanollösung, gepuffert im pH-Bereich von 10 - 14, aufgenommen, wovon 2 ml zur Befeuchtung eines Filters angewandt und die verbleibenden 5 ml in einen zweiten Behälter filtriert werden. Dann werden 0,2 ml der verdünnten Schwerölprobe aus Behältnis 1 in den zweiten Behälter übergeben. Der zweite Behälter wird nun für 30 Sekunden mittels des Vortexmischers vermischt. Aus dem zweiten Behälter werden dann mittels einer Kolbenspritze 4 ml der Mischung aufgenommen. Diese 4 ml werden durch den zuvor benetzten Filter in ein drittes Behältnis gegeben. Der dritte Behälter wird nun in ein Photometer gestellt, wobei er mit einer UV-Diode mit 365 nm Wellenlänge be- und durchstrahlt wird. Die resultierende Änderung der Strahlung kann bei 470 nm ausgewertet und über eine elektronische Schaltung an ein Anzeigegerät übergeben werden, oder es wird ein Protokoll mit den gemessenen Werten erstellt. Der gefundene Wert entspricht dem Gehalt an Aluminium in der Schwerölprobe und repräsentiert auch den Gesamtgehalt an Catfines.

## Patentansprüche

1. Verfahren zur Ermittlung des Gehalts an Catalyst Fines in einer Probe eines Schweröls, insbesondere eines Kraftstoffes für Schiffsmotoren, mittels folgender Schritte:
a) aus dem Schweröl wird eine Probe entnommen,
b) die Probe wird in einem ersten Behälter mit einem ersten Reagenz aus hauptsächlich Kohlenwasserstoffe enthaltenden Lösungsmittel verdünnt und intensiv vermischt,
c) ein Teil der Mischung des ersten Behälters wird in einen eine Mischung aus Methanol und KOH-Ethanol als ein zweites Reagenz enthaltenden zweiten Behälter eingebracht und damit intensiv vermischt,
d) aus dem zweiten Behälter wird ein Teil des Inhalts entnommen und über einen Filter in einen dritten Behälter überführt,
e) im dritten Behälter erfolgt die Feststellung des Gehalts an Catalyst Fines in der Probe durch Ermittlung der Stärke der sich ergebenden Fluoreszenzstrahlung bei UV-Lichtanregung
f) die sich zeigende Fluoreszenzstrahlung wird visuell oder mit Hilfe eines Photometers erfasst und bewertet.

2. Verfahren nach Anspruch 1, **dadurch gekennzeichnet, dass** das erste Reagenz ein Verdünner in Form eines aliphatische Kohlenwasserstoffe enthaltenden Universalkaltreinigers ist.

3. Verfahren nach Anspruch 1, **dadurch gekennzeichnet, dass** das zweite Reagenz eine 50:50 Mischung aus Methanol und KOH-Ethanol ist.

4. Verfahren nach Anspruch 1, **dadurch gekennzeichnet, dass** die Entnahme aus dem zweiten Behälter mittels einer Kolbenspritze erfolgt, deren Inhalt durch einen mit der Mischung aus Methanol und KOH-Ethanol benetzten Filter abgegeben wird.

5. Verfahren nach Anspruch 1, **dadurch gekennzeichnet, dass** die Vermischung gemäß Schritt c) mittels eines Vortexmischers bei Umgebungstemperatur durchgeführt wird.

6. Verfahren nach Anspruch 1, **dadurch gekennzeichnet, dass** die Vermischung gemäß Schritt c) mittels Ultraschall bei erhöhter Temperatur durchgeführt wird.

7. Verfahren nach Anspruch 1, **dadurch gekennzeichnet, dass** die Anregung durch UV-Strahlung mittels wenigstens einer UV-Diode bei 365 nm erfolgt.

8. Verfahren nach Anspruch 7, dadurch gekenntzeichnet, dass die Stärke der Fluoreszenzstrahlung bei 470 nm ermittelt wird.

9. Verfahren nach Anspruch 4, dadurch gekenntzeichnet, dass der Filter vor seiner Verwendung mit einer Mischung aus Methanol und KOH-Ethanolbei pH-Stabilisierung im Bereich von 10 -14 ph vorgespült wird.

10. Verfahren nach Anspruch 1, **dadurch gekennzeichnet, dass** in Schritt b) Schweröl (HFO) mit Verdünner in Form eines Universalkaltreinigers im Volumenverhältnis 1:10 für 2 - 4 Minuten im ersten Behälter mittels eines Vortexmischers gemischt wird, dass in Schritt c) 5 ml des zweiten Reagenz im zweiten Behälter mit 25 ml der Mischung aus dem ersten Behälter mittels eines Vortexmischers für 20 - 40 Sekunden vermischt werden, dass in Schritt d) 3 - 5 ml entnommen und gefiltert werden und das Filtrat in den dritten Behälter überführt wird, in dem die Ermittlung der Fluoreszenzstrahlung gemäß Schritt f) erfolgt.

11. Verfahren nach Anspruch 1, **dadurch gekennzeichnet, dass** das in Schritt d) aus dem zweiten Behälter entnommene Filtrat auf einen pH-Wert von 10 - 14 eingestellt wird.

## Claims

1. Method for determining the content of cat fines in a sample of a heavy oil, in particular a fuel for marine engines, by means of the following steps:
a) a sample is taken from the heavy oil,
b) the sample is diluted and intensively mixed in a first container with a first reagent consisting primarily of hydrocarbon-containing solvents,
c) some of the mixture in the first container is introduced into a second container containing a mixture of methanol and KOH ethanol as a second reagent, and is intensively mixed therewith,
d) some of the contents are removed from the second container and transferred into a third container via a filter,
e) the content of cat fines in the sample is ascertained in the third container by determining the intensity of the resulting fluorescence radiation upon UV light excitation,
f) the appearing fluorescence radiation is detected and evaluated visually or by using a photometer.

2. Method according to claim 1, **characterised in that** the first reagent is a diluting agent in the form of a universal cold cleaning agent containing aliphatic hydrocarbons.

3. Method according to claim 1, **characterised in that** the second reagent is a 50:50 mixture of methanol and KOH ethanol.

4. Method according to claim 1, **characterised in that** the removal from the second container takes place by means of a piston syringe, the contents of which are dispensed through a filter wetted with the mixture of methanol and KOH ethanol.

5. Method according to claim 1, **characterised in that** the mixing according to step c) is carried out using a vortex mixer at ambient temperature.

6. Method according to claim 1, **characterised in that** the mixing according to step c) is carried out using ultrasound at an increased temperature.

7. Method according to claim 1, **characterised in that** the excitation by UV radiation takes place using at least one UV diode at 365 nm.

8. Method according to claim 7, **characterised in that** the intensity of the fluorescence radiation is determined at 470 nm.

9. Method according to claim 4, **characterised in that** the filter is rinsed before use with a mixture of methanol and KOH ethanol when the pH stabilises to within the range of 10 to 14 pH.

10. Method according to claim 1, **characterised in that**, in step b), heavy oil (HFO) is mixed with a diluting agent in the form of a universal cold cleaning agent at a volume ratio of 1:10 for 2 to 4 minutes in the first container by means of a vortex mixer, **in that**, in step c), 5 ml of the second reagent is mixed in the second container with 25 ml of the mixture from the first container by means of a vortex mixer for 20 to 40 seconds, **in that**, in step d), 3 to 5 ml is removed and filtered and the filtrate is transferred into the third container, in which the fluorescence radiation is determined according to step f).

11. Method according to claim 1, **characterised in that** the filtrate removed from the second container in step d) is adjusted to a pH of 10 to 14.

## Revendications

1. Procédé pour déterminer la teneur en fines particules catalytiques dans un échantillon d'huile lourde, en particulier de carburant pour moteurs de bateau, à l'aide des étapes suivantes :
a) un échantillon est prélevé dans l'huile lourde,
b) l'échantillon est dilué et mélangé énergiquement, dans un premier récipient, avec un premier réactif composé de diluant contenant principalement des hydrocarbures,
c) une partie du mélange du premier récipient est amenée dans un deuxième récipient qui contient un mélange de méthanol et de KOH-éthanol, comme réactif, et est mélangée énergiquement avec ce mélange,
d) une partie du contenu est prélevée dans le deuxième récipient et est transférée, en passant par un filtre, dans un troisième récipient,
e) dans le troisième récipient a lieu la constatation de la teneur en fines particules catalytiques dans l'échantillon, par détermination de l'intensité du rayonnement fluorescent produit lors d'une excitation par une lumière UV,
f) le rayonnement fluorescent qui apparaît est détecté et analysé visuellement ou à l'aide d'un photomètre.

2. Procédé selon la revendication 1, **caractérisé en ce que** le premier réactif est un diluant sous forme de purificateur universel à froid contenant des hydrocarbures aliphatiques.

3. Procédé selon la revendication 1, **caractérisé en ce que** le deuxième réactif est un mélange 50:50 de méthanol et de KOH-éthanol.

4. Procédé selon la revendication 1, **caractérisé en ce que** le prélèvement dans le deuxième récipient se fait à l'aide d'une seringue dont le contenu passe à travers un filtre imprégné du mélange de méthanol et de KOH-éthanol.

5. Procédé selon la revendication 1, **caractérisé en ce que** le mélange de l'étape c) est réalisé à l'aide d'un mélangeur Vortex à température ambiante.

6. Procédé selon la revendication 1, **caractérisé en ce que** le mélange de l'étape c) est réalisé par ultrasons à une température accrue.

7. Procédé selon la revendication 1, **caractérisé en ce que** l'excitation par un rayonnement UV se fait à l'aide d'au moins une diode UV à 365 nm.

8. Procédé selon la revendication 7, **caractérisé en ce que** l'intensité du rayonnement fluorescent est déterminée à 470 nm.

9. Procédé selon la revendication 4, **caractérisé en ce que** le filtre, avant d'être utilisé, est pré-rincé avec un mélange de méthanol et de KOH-éthanol avec une stabilisation du pH dans la plage de 10-14 ph.

10. Procédé selon la revendication 1, **caractérisé en ce que** lors de l'étape b), l'huile lourde (HFO) est mélangée dans le premier récipient, à l'aide d'un mélangeur Vortex, avec un diluant sous forme de purificateur universel à froid avec un rapport de volume de 1:10 pendant 2-4 minutes, **en ce que** lors de l'étape c) 5 ml du deuxième réactif sont mélangés pendant 20-40 secondes dans le deuxième récipient avec 25 ml du mélange provenant du premier récipient, à l'aide d'un mélange Vortex, **en ce que** lors de l'étape d), 3-5 ml sont prélevés et filtrés, et le filtrat est transféré dans le troisième récipient, où a lieu la détermination du rayonnement fluorescent de l'étape f).

11. Procédé selon la revendication 1, **caractérisé en ce que** lors de l'étape d), le filtrat prélevé dans le deuxième récipient est amené à une valeur pH de 10-14.
